# EUROPEAN PATENT APPLICATION

(11) **EP 2 798 994 A1**
(43) Date of publication of application: **05.11.2014**
(21) Application number: 13166351.0
(22) Date of filing: 03.05.2013
(51) Int. Cl.: A47L 15/42

(54) **Washing/disinfecting installation**

(71) Applicant: Getinge Disinfection AB, 351 15 Växjö (SE)
(72) Inventor: Lagerkvist, Carl-Johan, 352 20 Växjö (SE)
(74) Representative: Hjalmarsson, Magnus Axel

(57) **Abstract**

The present disclosure relates to a washing/disinfecting installation with an installation structure and a number of washer/disinfectors (3, 5, 7, 9). The washer/disinfectors are arranged in a row (11), and the installation structure comprises vertical elements (29, 31) at each side of a washer/disinfector for carrying the washer/disinfector. Holding devices (43), attached to the vertical elements, carry a slider (45, 49) on which the washer/disinfector is loaded, being slideable in a direction perpendicular to the row, such that the side surfaces (17, 19) of said washer/disinfector may become accessible for maintenance measures.

## Description

### Technical field

The present disclosure relates to a washing/disinfecting installation comprising an installation structure and a plurality of washer/disinfectors arranged essentially in parallel in said installation structure thereby forming a row of washer/disinfectors. The installation has a soiled side on one side of the row and a clean side on the other, wherein goods to be cleaned may fed to the washer/disinfectors through washer/disinfector input openings at the soiled side, and cleaned goods may be fetched from washer/disinfector output openings at the clean side.

### Background

Such an installation is disclosed e.g. in EP-1237460-B1, where as an example six washer/disinfectors are placed in parallel, and an automatic loading system feeds goods to the washer/disinfectors.

One general problem with such an installation is how to accomplish improved maintenance and service, that can be carried out easier and more quickly in order to achieve a higher overall capacity.

One object of the present disclosure is therefore to obtain an installation of the initially mentioned kind with improved maintenance features.

### Summary

This object is achieved by an installation as defined in claim 1. More specifically, in an installation of the initially mentioned kind, the installation structure comprises vertical elements at each side of at least one washer/- disinfector for carrying the washer/disinfector in the installation structure. A lower section of the vertical element comprises a first and a second holding device, wherein the first and second holding devices of the vertical elements at each side of said at least one washer/disinfector carry a slider on which said at least one washer/disinfector is loaded. The slider is slideable in a direction substantially perpendicular to the row of washer/disinfectors, such that the sides of said washer/disinfector may become accessible for maintenance measures.

In this way maintenance can be carried out more easily on the washer/disinfector, as the side surfaces of the washer/disinfector readily becomes available. A large portion of the front and back surfaces of each washer/disinfector will be occupied by the washer/disinfector doors. However, interior features such as for instance pumps and control electronics can be readily accessed by sliding the washer/disinfector out of the installation to some degree and removing a side wall or opening doors provided in the side wall.

A holding device may be vertically adjustable in relation to the vertical elements. This makes possible to adjust the height of a corner of the washer/disinfector so as to compensate for washer/disinfector production tolerances. This simplifies the operation of automatic devices that are used to load goods to or unload goods from the washer/disinfectors in the installation.

Each vertical element may comprise a first and a second upright, interconnected by means of a lower bar, and the holding devices may then be suspended by the lower bar. If such a lower bar is provided, the holding device may be made vertically adjustable by means of a bolt extending through the holding device , the end of the bolt resting on the lower bar. The holding device may further comprise a pin which extends downwards into a hole or slit in the lower bar, in order to keep the holding device in place.

The lower bar may further rest on adjustable feet in order to compensate for the floor being an uneven surface.

A washing/disinfecting installation of the above mentioned kind may be provided with moveable a dish holder, which is conveyed by a traveller to selected washer/disinfectors to load goods from the dish holder and into the washer/disinfector.

### Brief description of the drawings

Fig 1A illustrates schematically a top view of a washing/disinfecting installation.
Fig 1 B illustrates the installation in fig 1A when maintenance is carried out on an associated washer/disinfector.
Fig 2 shows a part of an installation structure, which part is capable of carrying a washer/disinfector.
Fig 3 shows a detail of the structure in fig 2.
Fig 4 shows a cross-section through a slider device in a retracted position.
Fig 5 shows a miniture of the slider in fig 4 in an extended position.
Fig 6 shows an enlarged portion of theslider in fig 5.
Fig 7 shows a perspective view of a washing/disinfecting installation with one washer/disinfector pulled out for maintenance.

### Detailed description

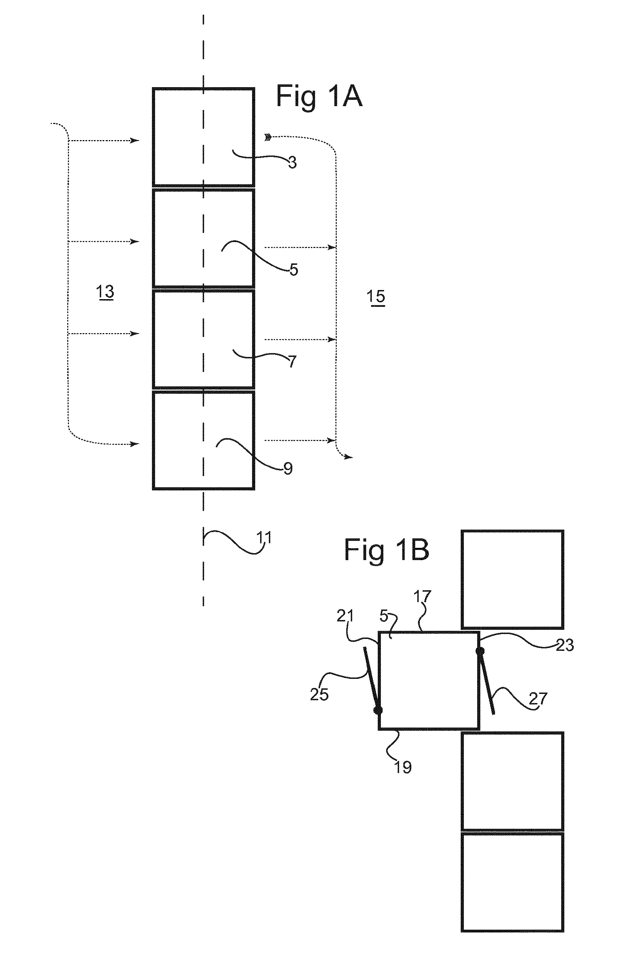

The present disclosure relates to a washing/disinfecting installation 1 as is schematically illustrated in fig 1A. This installation has a plurality of washer/disinfectors 3-9 arranged essentially in parallel in a row 11. The wording parallel here indicates that the goods to be cleaned can flow in parallel through the washer/disinfectors as indicated by arrows in the drawing.

Typically, there may be defined a soiled side 13 on one side of the row 11 of washer/disinfectors, and a clean side 15 on the other. This is very advantageous for instance in a washing/disinfecting installation in a hospital. The goods to be cleaned is then fed to the washer/disinfectors from the soiled side 13, and, when cleaned, the goods may be outputted on the clean side 15, given that at least one of the washer/disinfectors is provided with an opening at both the soiled and the cleaned side. In this way, the cleaned goods is separated from the goods to be cleaned as the row of washer/- disinfectors acts as a separating wall between those categories of goods.

Such an installation may be provided with automatic feeding of soiled goods and automatic fetching of cleaned goods as described e.g. in EP-1237460-B1, where a dish holder is conveyed by a traveller to a selected washer/disinfector to load goods from the dish holder and into the washer/- disinfector for processing. The traveller is supported by a track which extends along the row of washer/disinfectors.

The present disclosure describes an installation of the initially mentioned kind where maintenance of the associated washer/disinfectors is made substantially easier. This is accomplished by making a washer/disinfector 5 slideable in a direction perpendicular to the row 11, such that the side surfaces 17, 19 of the washer/disinfector become accessible for maintenance, as is illustrated schematically in fig 1 B.

This is advantageous, since most of the front and back surfaces 21, 23 of the washer/disinfector are covered by the washer/disinfector doors 25, 27. Accessing components inside the washer/disinfector, except for in the washer/disinfector cavity where the goods is placed during processing, is therefore difficult from the front and back surfaces 21, 23.

By accessing the side surfaces 17, 19, as illustrated, maintenance can be carried out more easily. Interior features, e.g. pumps or control electronics, can thus be readily accessed by sliding the washer/disinfector out of the installation and e.g. removing a side wall or opening provided doors (not shown) in the side wall.

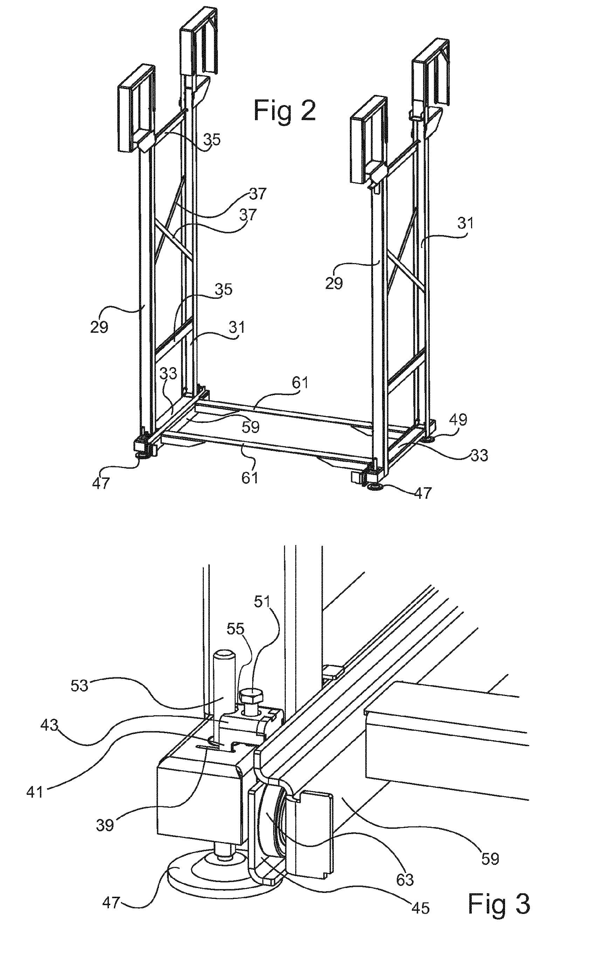

Fig 2 shows a part of an installation structure, being capable of carrying a washer/disinfector. Fig 3 shows a part of a lower section of the structure in fig 2. The installation structure comprises vertical elements, each comprising a first 29 and a second 31 upright, located at each side of a washer/disinfector (not shown). The pair of uprights at each side of a washer/disinfector are interconnected by a lower horizontal bar 33 in the installation structure. Each set of first and second uprights may further be interconnected by one or more upper bars 35 as well as by cross braces 37.

Close to the floor, typically on the lower horizontal bar 33, there is provided first and a second holding devices as is shown in more detail in fig 3. The lower horizontal bar 33 may thus be provided with a slit 39 or hole into which a pin 41 of a first holding device 43 extends. To this holding device 43, a first end of a fixed slider bar 45 may be connected, typically welded.

Each lower horizontal bar 33 rests on front 47 and rear 49 feet, which may be adjustable, e.g. by being threaded as is well known per se. This allows the feet extensions to be adjusted to compensate for the floor surface being uneven in any way. This allowes the horizontal bars 33 to be carefully lined up such that they extend in a common horizontal plane along the row of washer/disinfectors.

Further, it is desirable to adjust the vertical position of the holding device 43 in relation to the lower bar 33. This may, as shown in fig 3, be arranged by providing a bolt 51, threaded in the holding device, such that the bolt takes up the vertical load of the holding device 43, and the end of the bolt rests on the horizontal, lower bar 33. As shown in fig 3, the slit 39 may be long enough to accommodate pins 41 of two holding devices 43, one at each lateral side of the lower bar. An upright pin 53, centrally located at the lower bar end, may together with a notch 55 in each holding device 43 serve to position the holding device pin 41 correctly in the slit 39, and further stabilizes the holding device 43 in the elongated direction of the lower bar 33. The vertical position of the holding device 43 in relation to the lower bar 33 may be adjusted by turning the bolt 51.

Thanks to the vertical position of the holding device 43 being adjustable in relation to the lower bar 33, it is possible to compensate for manufacturing tolerances of the washer disinfectors 3, 5, 7, 9. Such an adjustable holding device 43 may be located close to each bottom surface corner of the washer/disinfector.

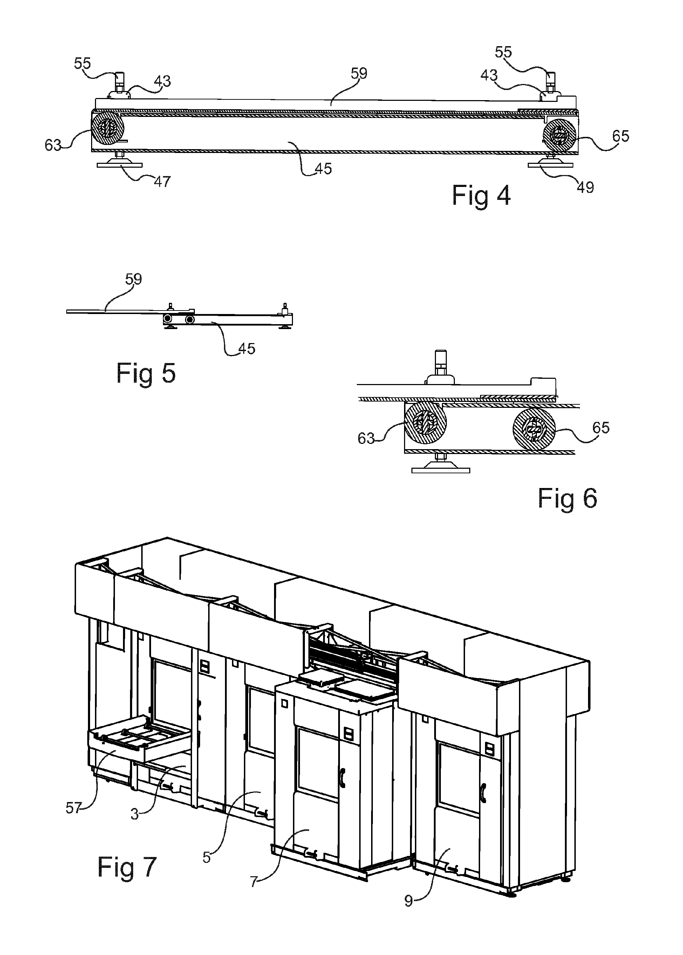

Even if the horizontal bars 33 are prefectly lined up in in an horizontal plane, the adjustable feet 47, 49 compensating for any deficiencies in the floor surface, manufacturing tolerances of the washer disinfectors may still cause e.g the washer disinfector openings to be disposed at slightly different heigts over the floor. The compensation for such tolerances, by adjusting the vertical position of the holding devices 43, makes easier and more reliable e.g. the operation of a dish holder 57 (cf. fig 7) which is conveyed by a traveller to selected washer/disinfectors to load goods from the dish holder and into the washer/disinfector for processing. Of course, those tolerances could instead be reduced until this adjustable feature would no longer be needed, but at the price of a higher washer/disinfector production cost.

As mentioned, the holding devices 43, carry a fixed slider bar 45. Each fixed slider bar 45 is slideably connected to a movable slider bar 59. As shown in fig 2, the movable slider bars 59 on each side of the space intended for a washer/disinfector are interconnected by two beams 61, one front and one rear beam as seen from the direction in which goods enter the washer/- disinfectors. The two beams 61, together with the slidable bars 59, form a slideable platform on which the washer/disinfector may be mounted.

The washer/disinfector thereby becomes slideable in a direction perpendicular to the row of washer/disinfectors, such that its sides become accessible for maintenance measures.

Fig 4 shows a cross-section through a slider device in a retracted position. The cross section is taken through the axial centre, along the rotation axis, of a front wheel/bearing 63 which is also visible in fig 3. The slideable bar 59 is supported by this front bearing 63, the centre portion of which is attached to the fixed bar 45 and the periphery of which rests against the slideable bar 59. The slideable bar 59 is further supported by a rear bearing 65, the centre portion of which is attached to the slideable bar 59 and the periphery of which rests against the fixed bar 45. Thereby, the slideable bar can be extended out of the installation structure to an extended position as is indicated in the miniture in fig 5. It may be preferred to arrange the slideable bar 59 to be inclined slightly upwards in this state when unloaded by a washer/disinfector so as to compensate for bending of the slideable bar 59 when cantilevered in this position and being loaded. As illustrated in fig 5 and more clearly in the enlarged portion in fig 6, the rear wheel/bearing 65 in this state has travelled most of the way towards the front wheel/bearing 63.

Fig 7 shows a perspective view of a washing/disinfecting installation with one washer/disinfector 7 pulled out for maintenance. Before extending the washer/disinfector out of the row, the aforementioned dish holder 57 has been positioned in front of another washer/disinfector 3 that will remain in the row. The side walls of the extended washer/disinfector is now readily available for maintenance purposes.

The present invention is not limited to the above described embodiment and may be varied and altered in different ways within the scope of the appended claims.

## Claims

1. A washing/disinfecting installation comprising an installation structure and a plurality of washer/disinfectors (3, 5, 7, 9) arranged essentially in parallel in said installation structure thereby forming a row (11) of washer/- disinfectors, the installation having a soiled side (13) on one side of the row and a clean side (15) on the other side, wherein goods to be cleaned may fed to the washer/disinfectors through washer/disinfector input openings at the soiled side, and cleaned goods may be fetched from washer/disinfector output openings at the clean side, **characterised by** the installation structure comprising vertical elements (29, 31) at each side of at least one washer/- disinfector for carrying the washer/disinfector in the installation structure, a lower part of a vertical element comprising a first and a second holding device (43), wherein the first and second holding devices of the vertical elements at each side of said at least one washer/disinfector carry a slider (45, 59) on which said at least one washer/disinfector is loaded, the washer/disinfector thereby being slideable in a direction substantially perpendicular to said row, such that the side surfaces (17, 19) of said washer/disinfector may become accessible for maintenance measures.

2. A washing/disinfecting installation according to claim 1, wherein at least one holding device (43) is vertically adjustable in relation to the vertical elements.

3. A washing/disinfecting installation according to claim 1 or 2, wherein the vertical element comprises a first (29) and a second (31) upright, interconnected by means of a lower bar (33), the holding devices (43) being suspended by said lower bar.

4. A washing/disinfecting installation according to claim 2 and 3, wherein said at least one holding device (43) comprises a pin (41) which extends downwards into a hole (39) in the lower bar (33), and the holding device is made vertically adjustable by means of a bolt (51) extending through the holding device.

5. A washing/disinfecting installation according to claim 3 or 4, wherein the lower bar (33) rests on adjustable feet (47, 49).

6. A washing/disinfecting installation according to any of the preceding claims, wherein the installation comprises a dish holder (57), which is conveyed by a traveller to selected washer/disinfectors to load goods from the dish holder and into the washer/disinfector.
